Europäisches Patentamt

European Patent Office

Office européen des brevets

(19)

(11) Veröffentlichungsnummer: **0 203 027**
**B1**

(12) **EUROPÄISCHE PATENTSCHRIFT**

(45) Veröffentlichungstag der Patentschrift: **23.05.90**

(51) Int. Cl.⁵: **A 61 N 1/365,** A 61 B 5/02

(21) Anmeldenummer: **86730032.9**

(22) Anmeldetag: **24.02.86**

(54) **Bedarfsschrittmacher mit physiologischer Steuerung.**

(30) Priorität: **22.02.85 DE 3506789**

(43) Veröffentlichungstag der Anmeldung:
**26.11.86 Patentblatt 86/48**

(45) Bekanntmachung des Hinweises auf die
Patenterteilung:
**23.05.90 Patentblatt 90/21**

(84) Benannte Vertragsstaaten:
**AT CH DE FR GB LI NL SE**

(56) Entgegenhaltungen:
**EP-A-0 080 348**
**EP-A-0 118 306**
**WO-A-8/23782**
**DE-A-3 240 430**
**DE-A-3 422 112**

(73) Patentinhaber: **BIOTRONIK Mess- und
Therapiegeräte GmbH & Co Ingenieurbüro
Berlin
Sieversufer 8
D-1000 Berlin 47 (DE)**

(72) Erfinder: **Schaldach, Max, Prof. Dr.-Ing.
Turnstrasse 5
D-8520 Erlangen (DE)**

(74) Vertreter: **Christiansen, Henning, Dipl.-Ing.
Patentanwalt CHRISTIANSEN Pacelliallee 43/45
D-1000 Berlin 33 (DE)**

**Beschreibung**

Die Erfindung betrifft einen Herzschrittmacher der im Oberbegriff des Anspruchs 1 angegebenen Art.

Es sind physiologisch gesteuerte Herzschrittmacher bekannt, bei denen das Escape-Intervall durch einen vom Körper abgeleiteten Parameter gesteuert wird. In diesem Zusammenhang ist in "Biomedizinische Technik", 20, 1975, Seiten 225, 226, in dem Aufsatz: "Ein Herzschrittmacher mit belastungsabhängiger Frequenzregulation" die Steuerung durch die Respirationsrate beschrieben, während in der US-PS Nr. 4 467 807 ein Schrittmacher mit Steuerung der Grundrate durch die Sauerstoffsättigung dargestellt ist.

Physiologische Steuerung bedeutet dabei eine Beeinflussung des Schrittmachers zur Anpassung der Leistungsfähigkeit des Herzens entsprechend dem augenblicklichen Bedarf, d.h. insbesondere bezüglich seiner Grundrate, die unabhängig ist von der bisherigen patientenabhängigen Steuerung durch im Herzen abgeleitete elektrische Signale, welche allein eine Vermeidung der Konkurrenz von stimulierten Impulsen mit Spontanaktionen zum Ziele hat.

Bei derartigen Schrittmachern ist nachteilig, daß die Vorgabe der Abhängigkeit des Escape-Intervalls vom physiologischen Parameter entweder willkürlich vorgegeben oder aber vom behandelnden Arzt in einer langwierigen Versuchsreihe an den individuellen Patienten angepaßt werden muß.

Der im Anspruch 1 angegebenen Erfindung liegt die Aufgabe zugrunde, die patientenindividuelle Anpassung zu vereinfachen und so zu ermöglichen, daß neben einer anfänglichen Anpassung die individuellen Stimulationsbedürfnisse auch fortwährend einer Aktualisierung des Betriebsverhaltens des Schrittmachers entsprechend dem sich ändernden Stimulationsbedürfnis vorgenommen wird.

Die Erfindung beruht auf der Erkenntnis, daß die grundsätzliche Abhängigkeit der Herzrate von dem zur Steuerung des Stimulations-Intervalls gewählten physiologischen Parameter unabhängig davon, ob die Herzaktionen spontan oder stimuliert erfolgen, bei wechselnder körperlicher Belastung, d.h. unterschiedlichem Leistungsbedarf des Herzens im wesentlichen erhalten bleibt, so daß nach dem entsprechendem "Lernvorgang" dann derjenige Herzfrequenzbereich, der bei der täglichen Belastung des Patienten ohne Schrittmacherintervention durchlaufen wird, auch bei Ausfall von Spontanaktionen als Stimulationsbereich zur Verfügung steht.

Für den Fall, daß bei der Anwendung für einen Demand-Schrittmacher ein physiologischer Parameter gewählt wird, der selbst von der Art der Kammeraktionen (spontan oder stimuliert) abhängt, ist in einer bevorzugten Weiterbildung der Erfindung vorgesehen, daß dieser Unterschied durch eine programmierbare-ggf. von der Herzfrequenz abhängige-Korrekturgröße kompensiert wird, welche in einem separaten Speicher festgehalten ist. Das entprechende Ergebnis wird auch erzielt, wenn in zwei unterschiedlichen Speicherbereichen jeweils das Escape-Intervall definierende Signalzustände festgehalten sind, welche in Abhängigkeit davon, ob die vorangehende Herzaktion spontan oder stimuliert erfolgte, entsprechend unterschiedlich abgerufen werden. Auf diese Weise ist es möglich, das aufgrund eines physiologischen Parameters ermittelte Escape-Intervall auch im Falle von Spontan-Aktionen "mitzuführen", so daß auch beim Ausfall von Spontan-Aktionen unter Belastung dem patienten eine adäquate Stimulationsrate zugutekommt.

Günstigerweise wird in einem entsprechenden Speicher die letzte zu dem aktue en Wert des Parameters gehörige Spontanfrequenz festgehalten und nach Ausfall von Spontan-Aktionen mit einem dieser Frequenz entsprechenden Escape-Intervall stimuliert. Dabei ist das Escape-Intervall um eine Differenzzeit länger zu bemessen als die der Spontanfrequenz entsprechende Rate, um einem physiologisch bedingten Frequenzabfall folgen zu können. Dieser Differenzwert ist derart bemessen, daß die physiologisch annehmbaren Frequenzabsenkungen (d/dt) ohne Schrittmacherintervention erfolgen können.

Der Betrieb des Schrittmachers teilt sich also in eine "Eichphase" bei auftretenden Spontanaktionen, bei denen die Zuordnung des später für die Steuerung der Stimulationsrate heranzuziehenden Parameters zur natürlichen Herzfrequenz ermittelt wird. Beim späteren Ausfall von Spontanaktionen wird dann auf diese gespeicherten Werte zurückgegriffen und die Stimulation entsprechend dem natürlichen Herzrhythmus fortgesetzt, wobei diese Stimulationsrate von der natürlichen Rate abweichen kann. Dafür gibt es drei Gründe: Entweder wird die Stimulationsrate jeweils niedriger gewählt als die entsprechende natürliche Rate, um nicht unnötig mit künstlichen Stimulationsimpulsen zu intervenieren oder aber sie wird größer gewählt (entsprechend dem bekannten mit festliegender Stimulationsrate arbeitenden Hystereseschrittmacher) oder aber sie wird adequat angepaßt, wenn der gewählte physiologische Parameter von Aktivierungszustand des Herzens selbst abhängig ist (z.B. QT-Intervall) und insoweit eine Kompensation erforderlich ist.

Bei einer anderen bevorzugten Ausführungsform wird bei einer definiert einschaltbaren "Eichphase" eines mit der erfindungsgemäßen Steuerung versehenen Demandschrittmachers die künstliche Stimulationsrate besonders herabgesetzt, um das natürliche Herzverhalten und den physiologischen Verlauf der Stimulationsrate nur möglichst wenig durch Stimulationsintervention zu unterbrechen.

Gemäß einer anderen vorteilhaften Ausführung der Erfindung lassen sich solche Frequenzbereiche innerhalb des für die Stimulation in Betracht kommenden Bereiches der Herzfrequenzen von der Stimulation ausnehmen-wie durch separate Untersuchungen zu ermitteln ist -, so daß im Falle des Ausfalls von Spontan-Aktionen mit der Stimulation bei einem höheren Wert fortgefahren wird als es dem aktuellen Wert des das Escape-Intervall steuernden physiologischen Parameters entspräche. Entsprechend der in dem auszunehmenden Frequenzbereich vorhandenen Leistungsfähigkeit des Herzens läßt sich vorsehen, daß bei vorhandenen Spontan-Aktionen das Escape-Intervall zunächst entsprechend der Veränderung des physiologischen Parameters nachgeführt wird. Sobald derartige Spontan-Aktionen innerhalb des jeweils gültigen Escape-Intervalls aber ausfallen, erfolgt die Stimulation mit einer Frequenz oberhalb des auszunehmenden Frequenzbereiches, bis durch den physiologischen Parameter das Escape-Intervall wieder auf einen Wert gesetzt wird, der zu einer Frequenz unterhalb des auszunehmenden Frequenzbandes gehört. Im Falle einer stark verminderten Leistungsfähigkeit des Herzens im auszunehmenden Frequenzbereich läßt sich durch entsprechende Voreinstellung mittels externer programmierung festlegen, daß auch bei vorhandenen Spontan-Aktionen eine höhere Herzrate durch künstliche Stimulation erzeugt wird, wenn die Frequenz sich innerhalb des auszunehmenden Frequenzbereiches befindet. Das Durchlaufen der in Abhängigkeit von der Richtungssinn der Änderung des Parameters unterschiedlichen auszunehmenden Escape-Intervallbereiche erfolgt nach Art einer Hysteresekurve. Diesen Lösungen ist gemeinsam, daß der Verlauf der Abhängigkeit zwischen dem zur Steuerung herangezogenen physiologischen Parameter und der Schrittmacherrate ergänzend zu der sich gemäß der Erfindung ergebenden Abhängigkeit auch noch manuell individuell durch programmierung abgeändert werden kann.

Bei physiologischen Parametern, deren funktionaler Zusammenhang zur Herzfrequenz von der Art der Herzaktion (spontan oder stimuliert) abhängt, läßt sich die Differenz der zu einer aktuellen Stimulationsfrequenz gehörigen physiologischen Parameter jeweils für den Fall einer Spontan-Aktion und einer stimulierten Aktion dann ermitteln, wenn nach vorhandenen Spontan-Aktionen in den Stimulationszustand (oder umgekehrt) übergegangen wird.

Die einzelnen Lösungsgedanken sind auch unabhängig voneinander günstig verwendbar und stellen insoweit separat einzuschlagende Lösungswege dar.

Zur Anwendung eignen sich bevorzugt die physiologischen Parameter "QT-Intervalle", "Systolische Intervalle" oder durch Impedanzmessung im Herzen (elektroplethysmographisch) aufgenommene Impulse.

Das hier beschriebene Verfahren läßt sich auf in verschiedenen Modes arbeitende Schrittmacher anwenden, wobei der Einkammerschrittmacher zunächst bevorzugt wird. Bei einem Wechsel von der ventrikulären zu AV-sequentiellen Stimulation ist zu berücksichtigen, daß in beiden Fällen wegen des unterschiedlichen Grads der Kammerfüllung bzw. der vom Stimulationsmode abhängigen systolischen Intervalle getrennte funktionale Abhängigkeiten zu berücksichtigen sind, die in separaten Speichern festgehalten werden müssen. Da im übrigen die Verhältnisse analog sind, kann — jeweils bezogen auf einander entsprechende Stimulationsfrequenzen — beliebig von einem Mode zum anderen gewechselt werden, wenn die Verhältnisse es erfordern.

Die automatische Anpassung der Stimulationsfrequenz läßt sich durch programmierung bezüglich der in Betracht kommenden Frequenzgrenzen beeinflussen. Bei der automatischen Adaption entsprechend einem "Selbstlernvorgang", der auch ohne Durchlaufen eines Belastungszyklus unter Beaufsichtigung eines Arztes aufgrund der sich bei der normalen Tätigkeit Belastungsänderungen möglich ist, würde der Steuerbereich für stimulierte Frequenzen demjenigen angepaßt, der jeweils unter Auftreten von Spontanaktionen durchlaufen wurde.

Die Funktion des auf die dargestellte Weise frequenzgesteuerten Schrittmachers ist für den behandelnden Arzt insbesondere bei Ausnutzung der Möglichkeiten der Zweiweg-Kommunikation jederzeit ohne weiteres einsichtig. Günstig ist dazu bei der externen Kommunikationseinheit ein LCD-Display vorzusehen, auf dem sich die funktionale Abhängigkeit von den systolischen Zeitintervallen und Stimulationsfrequenz (RR-Abstand), in den innerhalb des Schrittmachers gespeicherten Werte graphisch darbietet. Der Grad der sich hier ergebenden Linearität der erfaßten Abhängigkeiten bei einem oder mehreren Belastungszyklen in der der oben angegebenen Weise, geben dem Arzt in anschaulicher Weise Aufschluß über die Möglichkeiten des hier beschriebenen Verfahrens im Hinblick auf den jeweiligen Patienten. Durch Berücksichtigung von Krümmungen des sich darbietenden funktionalen Zusammenhangs lassen sich extern in entsprechenden dazu reservierten Speicherplätzen Vorzugsfrequenzen manuell programmieren, welche dem Arzt für den bestimmten Anwendungsfall günstig erscheinen. Durch die individuelle programmierbarkeit der funktionalen Abhängigkeit von Stimulationsfrequenz als Grundrate bei der Demandfunktion oder auch bei einem Schrittmacher ohne diese Eigenschaft kann verschiedensten Krankheitsbildern Rechnung getragen werden.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur 1 ein Blockschaltbild eines Ausführungsbeispiels des erfindungsgemäßen Herzschrittmachers,

Figur 1a ein Detail des Blockschaltbilds gemäß Figur 1,

Figur 2 ein Kommunikationsteil für die externe Abfrage und Steuerung des Schrittmachers durch den Arzt,

Figur 3 einen akustischen Meßwertaufnehmer in vergrößerter Wiedergabe im Schnitt.

Die Darstellung der Erfindung wird nachfolgend anhand eines Ausführungsbeispiels vorgenommen, daß aufgrund der Steuerung mittels des physiologischen Parameters "systolische Intervalle" arbeitet — es lassen sich aber auch sämtliche anderen zur "physiologischen Schrittmachersteuerung" bekannten Parameter entsprechend verwenden.

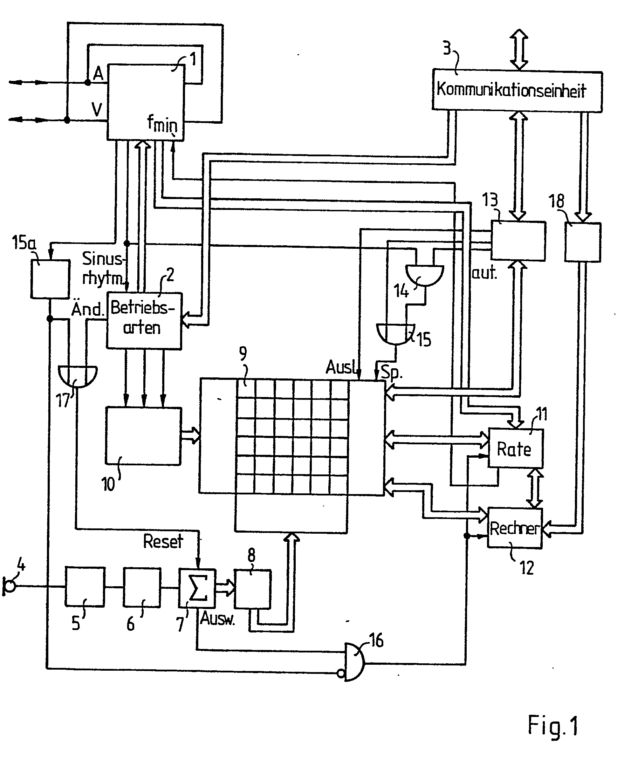

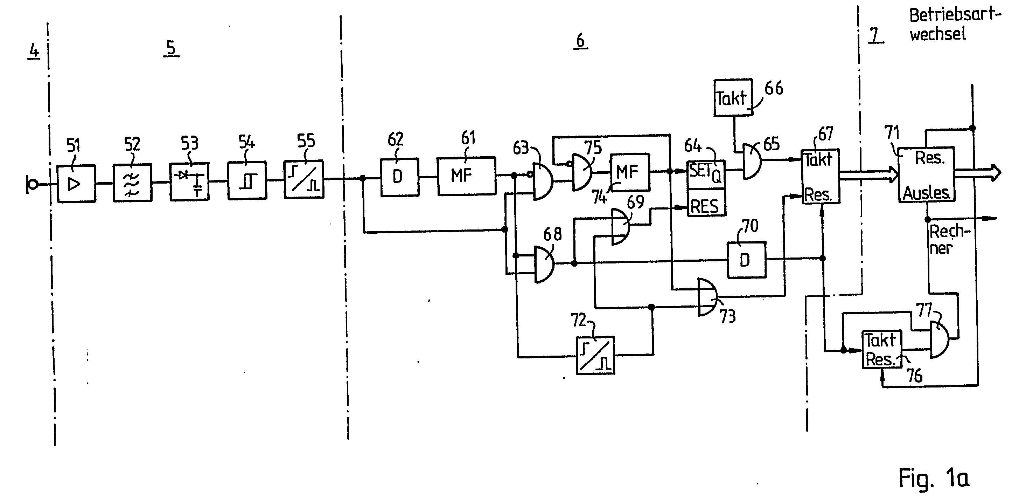

Das in Figur 1 wiedergegebene Blockschaltbild des Ausführungsbeispiels enthält einen konventionellen programmierbaren Atrium- und/oder Ventrikel-Schrittmacher mit Demand-Funktion. Die entsprechenden Ein- und Ausgänge zur Verbindung mit der Atrium- bzw. Ventrikel-Elektrode sind mit "A" und "V" gekennzeichnet. Der Betriebszustand des Steuerbausteins 1 für die Grundfunktionen des Schrittmachers wird durch eine Schalteinheit 2 für die Betriebszustände (Modes) des Schrittmachers bestimmt, welche über externe programmiermittel mittels einer Kommunikationseinheit 3 beeinflußbar ist. Durch die Schalteinheit 2 lassen sich also verschiedene Betriebszustände (VVI-, DAT-, DDD-Mode etc.) einstellen. Die bei den bisherigen multiprogrammierbaren Schrittmachern von außen einstellbare Grundrate oder Interventionsfrequenz läßt sich bei dem hier dargestellten Schrittmacher durch externe programmierung in den Betriebszustand "Physiologische Steuerung" durch im Körper aufgenommene Signale selbsttätig den Bedürfnissen des Patienten anpassen.

Als Besonderheit enthält der hier dargestellte Steuerbaustein somit einen Anschluß $f_{min}$ zur Beeinflussung der Grundfrequenz. Die übrigen in Figur 1 wiedergegebenen Baugruppen sind ebenfalls in dem implantierbaren Gehäuse des Schrittmachers zusammengefaßt.

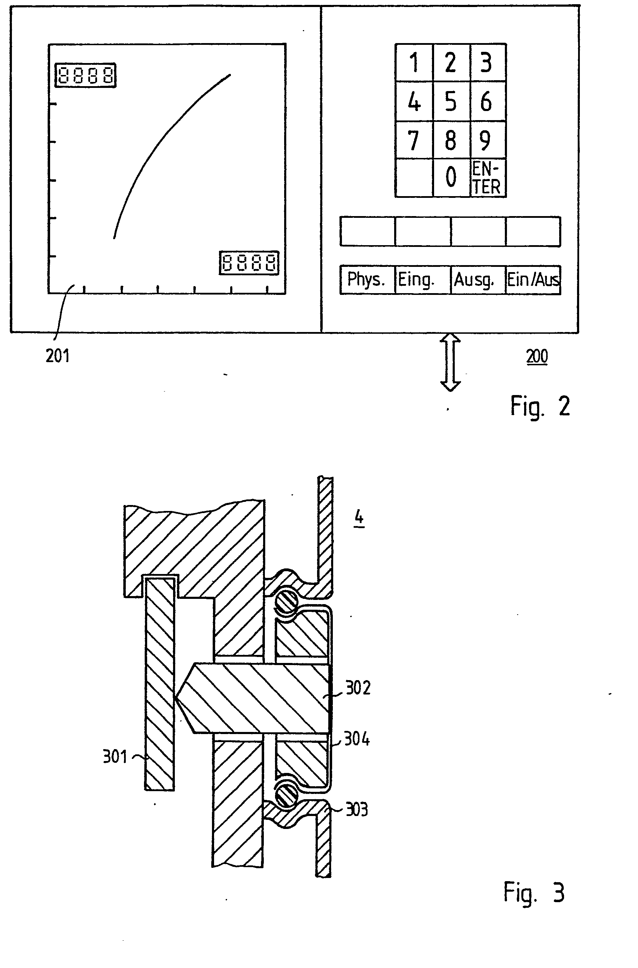

Die Grundrate, d.h. das Escape-Intervall, beginnend mit einer vorangegangenen stimulierten oder spontanen Atrium- bzw. Ventrikelaktion und endend mit dem Zeitpunkt, in dem bei ausfallender Spontanaktion im Atrium- bzw. Ventrikel stimuliert wird, ist durch die übrige dargestellte Anordnung veränderbar. Die Steuerung erfolgt in Abhängigkeit von innerhalb des Körpers des Patienten akustisch aufgenommenen Signalen, den linksventrikulären systolischen Intervallen. Diese Signale werden mittels eines Mikrofons 4 aufgenommen, welches als Körperschallempfänger ausgebildet ist und bevorzugt einen piezokeramischen Schallwandler enthält. Die Einzelheiten eines zur Ausführung der Erfindung geeigneten Schallaufnehmers werden anhand von Fig. 3 näher dargestellt. Das Ausgangssignal des Schallaufnehmers wird einem Verstärker 5 zugeleitet, welcher eine Ausfilterung der relevanten Frequenzbereiche vornimmt und einen Ausgangssignal abgibt, wenn Signale der ausgewählten Frequenzen für einen vorgegebenen Mindestzeitraum anstehen. In Betracht kommen vorzugsweise Frequenzen, wie sie bisher zur phonokardiologischen Untersuchung der systolischen Intervalle hernagezogen wurden.

Das Ausgangssignal der Stufe 5 wird einem Intervalldiskriminator 6 zugeleitet, welcher — gestartet durch die Vorderflanke eines ersten Eingangsimpulses — die Zeitdauer bis zu einem nachfolgenden Impuls ermittelt, wobei jeweils durch den ersten eingehenden Impuls ein Zurücksetzen erfolgt und die Auswertung auf ein vorgegebenes Zeitfenster begrenzt ist, nach deren erfolglosem Ablauf erneut ein Zurücksetzen der Meßeinrichtung vorgenommen wird. Erscheinen jedoch zwei aufeinanderfolgende Impulse innerhalb des Zeitfensters, so wird der zeitliche Abstand der Vorderflanken als digitales Signal ausgegeben und einem Mittelwertspeicher 7 zugeführt, welcher aus einer Anzahl von aufeinanderfolgenden Zeitintervallwerten einen Mittelwert bildet und diesen Mittelwert festhält.

Der Mittelwert wird bei einem Wechsel der eingestellten Betriebsart des Schrittmachers (auch durch externe programmierung) oder aber durch einen Wechsel des Stimulationszustandes (Übergang vom Betrieb mit Herzeigenaktion zum stimulierten Betrieb zurück etc.) zurückgesetzt, so daß eine Mittelwertbildung der aufeinanderfolgenden systolischen Intervalle nur für gleichartige Betriebszustände erfolgt.

Das Ausgangssignal des Mittelwertspeichers 7 wird in einem Adressdecoder 8 übertragen, wobei die vom Decoder ausgewerteten Adressen durch die gewählte Digitalisierung entsprechend der kleinsten Stufung der Digitalwerte gequantelt ist, so daß die Auflösung der aufzuwertenden Zeitintervalle jeweils die Differenz zweier benachbarter Speicheradressen bildet. Der Adreßdecoder bewirkt noch zusätzlich insofern eine Umformung der Ausgangssignal des Mittelwertspeichers 7, als daß von den digitalen Eingangswerten der Minimalwert eines aufzuwertenden systolischen Zeitintervalls subtrahiert und gegebenenfalls zusätzlich die Adresse des Speicherplatzes (innerhalb eines größeren Speicherbereiches) addiert wird. Der in Figur 1 dargestellte Speicher 9 ist matrixartig organisiert, wobei durch die systolischen Zeitintervalle die Spalten adressiert werden, während durch einen vom Betriebsartenspeicher 2 beeinflußten wei' teren Adreßdecoder 10 die Zeilen des matrixartig organisierten Speichers adressiert werden. Auf diese Weise wird erreicht, daß jeweils die systolischen Zeitintervalle nur für gleichartige Betriebszustände (Spontanaktionen, ventrikuläre oder AV-sequentielle Stimulation) festgehalten werden. Beim Übergang vom einen in den anderen Betriebszustand wird der Mittelwertspeicher 7 über seinen "Reset"-Eingang gelöscht und mit der Mittlung neu begonnen.

Der Speicher 9 wird bei einer Ausführung des Speichers mit kommerziell erhältlichen RAM-Elementen so ausgeführt sein, daß die Decoder 8 und 10 verschiedene Adressleitungen ansteuern, wobei beide Adressensignale eine gemeinsame binäre Adresse bilden, so daß die Adressenansteuerung mit derjenigen üblicher Mikroprozessorsysteme übereinstimmt. Die bei den adressierten Speicherwerten vorhandene Grundrate wird mit einem Grundratenspeicher 11 übertragen, in den sie als pufferspeicher übernommen werden, wobei dieser im Grundratenspeicher enthaltene Wert die minimale Stimulationsfrequenz für die Schrittmacherschaltung festlegt.

Auf diese Weise wird in Abhängigkeit von den ermittelten systolischen Zeitintervallen und der augenblicklichen Betriebsart des Schrittmachers eine minimale Stimulationsrate vorgegeben, welche den ermittelten systolischen Intervallen physiologisch entspricht. Durch die vorgenommene Tabellierung einer — wie nachfolgend zu beschreiben ist — vorher patientenindividuell ermittelten Abhängigkeit ist die Steuerung unabhängig von weiteren die systolischen Zeitintervalle beeinflussenden physiologischen Abhängigkeiten, sofern diese mit der Grundrate zusammenhängen. Wenn sich nämlich die ermittelten Intervalle ändern, so wird nach und nach eine Veränderung der Grundrate erfolgen, wobei das zur Einleitung der Veränderung aufgenommene Intervall nicht derjenigen Intervallzeit zu entsprechen braucht, welche zu der einzustellenden Zielfrequenz gehört. Sofern die bis zum Erreichen der Zielfrequenz zu berücksichtigende Änderung der Intervalle nicht das Vorzeichen wechselt. Hiermit wird die dargestellte Steuerung bei festgestellten Abweichungen die Stimulationsfrequenz (bedingt auch durch die Arbeitsweise des Mittelwertspeichers 7) langsam zu der neuen physiologisch bedingten Stimulationsfrequenz hinführen, wobei die im Verlaufe der Annäherung zu laufenden weiteren Intervalländerungen das Erreichen der endgültigen Zielfrequenz nicht stören, wenn der Zusammenhang zwischen aufgenommenem Zeitintervall und zugehöriger Schrittmacherrate eindeutig ist. Der Pufferspeicher für die Grundrate wird jeweils durch ein Signal "Auswertung" aktiviert, welches vom Mittelwertspeicher 7 abgeleitet wird und den Eingang eines neuen systolischen Intervallwertes signalisiert. Bei diesem Betrieb wird davon ausgegangen, daß sämtliche Frequenzwerte im Speicher 9 durch die Kommunikationseinheit 3 von außen übertragen wurden, wobei die Kommunikationseinheit den Schrittmacher über ein Steuerteil 13, welches der externen Programmierung unterliegt, in den Zustand "Auslesen" setzt, so daß jeweils bei der Adressierung durch die systolischen Zeitintervalle der im Speicher 9 vorhandene zugehörige Wert gelesen wird.

Bei einer Betriebsart, die durch das Steuerteil 13 über den Ausgang "Aut" gekennzeichnet wird, erfolgt bei Vorhandensein von Eigenaktionen (entsprechender Ausgang des Schrittmacherteils 1) ein Betrieb mit selbsttätiger Speicherung, der zu bestimmten systolischen Intervallen gehörigen Spontanfrequenz in den Speicher 9, so daß diese Signale bei später wieder notwendig werdender Stimulation mit herangezogen werden können. Der Abstand zwischen zwei aufgenommenen gleichartigen Spontanaktionen im Herzen (innerhalb eines physiologisch vertretbaren Bereichs) wird in diesem Fall vom Schrittmacherteil 1 in den Grundratenspeicher 11 übertragen und in den jeweils adressierten Speicherplatz eingelesen, der in diesem Fall über den Betriebsartenadressierer 2 Eigenaktionen zugeordnet ist. Dabei wird der Eingang "Speichern" des Speichers 9 über den Ausgang "Aut" des Steuerteils 13 und das Ausgangssignal "Eigenaktionen" 1 des Schrittmachers mittels eines UND-Gatters 14 verknüpft und der Eingang "Speichern" des Speichers 9 über ein ODER-Gatter 15 angesteuert.

Vom Schrittmacherteil 1 wird weiterhin aufgrund der dort aufgenommenen Signale aus dem Herzen eine Tachycardie- (bzw. Extrasystolen-) und Störungserkennung durchgeführt, die mittels einer entsprechenden Baugruppe 15 verarbeitet wird. Bei Auftreten von entsprechenden Zuständen erfolgt eine Sperrung der Verarbeitung der Ausgangssignale des Mittelwertspeichers 7 über den invertierenden Eingang eines in den entsprechenden Ausgang des Mittelwertspeichers 7 eingefügten UND-Gatters 16. Gleichzeitig wird über ein zusätzliches ODER-Gatter in der Rücksetz-Leitung des Mittelwertspeichers 7 dessen Zurücksetzen in den Ausgangszustand veranlaßt.

Bei der zuvor dargestellten Einspeicherung der aktuellen systolischen Intervalle bei Spontanaktionen erfolgt die Steuerung aufgrund der abgespeicherten Werte bei der Stimulation unter der Kontrolle eines zusätzlich vorgesehenen Rechners 12, der ausgelöst durch ein neues im Herzen aufgenommenes Intervallsignal, die zugehörige Grundrate im Speicher 11 aufgrund zusätzlicher Informationen und über die Kommunikationseinheit 3 eingegebener Vorgaben zusätzlich verändern kann, wobei Zugriff auf den Speicher 9 besteht. Diese Veränderung besteht insbesondere in der Anbringung eines Korrekturwertes, welcher bei der Steuerung der Grundrate des Schrittmachers unter Stimulationsbedingungen und Auswertung der entsprechenden Intervalle bei Spontanaktionen, die sich aufgrund der Änderung dieser Bedingungen für das Herz ergebenden Änderung der systolischen Intervalle berücksichtigt. Weiterhin kann eine Interpolation oder ein Ausgleich bei sich ergebenden Frequenzsprüngen bei benachbarten Speicherplätzen ausgeglichen werden. Außerdem lassen sich über die Kommunikationseinheit 3 programmierte Grenzwerte 18 für die Stimulationsfrequenzen vorgeben, welche bei der Nachführung der Grundrate des Schrittmachers nicht überschritten werden. Der entsprechende Grenzwertspeicher wird vom Rechner gelesen und bei Überschreitung des entsprechendes Wertes durch die Steuerung aufgrund der systolischen Intervalle eine Korrektur des im Grundratenspeicher 11 übernommenen Frequenzwertes bewirkt.

In Figur 1a ist der aus den Bausteinen 4 bis 7 bestehende Eingangsteil der Schaltung im Detail wiedergegeben. Dabei besteht die im Mikrofon 4 nachgeschaltete Verarbeitungsgruppe aus einem Eingangsverstärker 51 zur Heraufsetzung der Eingangssignale, einem Filter 52 zur Ausfilterung der für die systolischen Zeitintervalle charakteristischen Frequenzbereiche, einem Schmitt-Trigger 53, einem Gleichrichter und Siebglied 54, der Umwandlung der Frequenzsignale in Impulse einer Polarität, einem Schmitt-Trigger als Schwellwertdiskriminator und einem Impulsformer 55 zur Umwandlung der Vorderflanke des Ausgangsimpulses der Schaltung 54 in einen kurzen Steuerimpuls.

Der Zeitauswertungsteil 6 besteht aus einem monostabilen Multivibrator 61, dem das Ausgangssignal aus der Stufe 5 mittels eines Verzögerungsgliedes 62 um einige Millisekunden verzögert zugeleitet wird. Die Impulsdauer des monostabilen Multivibrators entspricht der maximal auszuwertenden systolischen Intervalldauer. Bei nicht gesetztem Monoflop 61 gelangt das Eingangssignal über ein UND-Gatter 63, dessen weiterer (invertierender) Eingang mit dem Ausgang des Monoflops 61 verbunden ist, zu dem "Setz"-Eingang eines Flip-Flops 64, welches über seinen Q-Ausgang ein UND-Gatter 65 freigibt, so daß ein vom einem Taktsignalgeber 66 erzeugtes Impulssignal zu dem entsprechenden Eingang eines Zählers 67 gelangt, so daß der Zähler entsprechend der abgelaufenen Zeit aufwärts zählt.

Der das systolische Intervall beendende Impuls gelangt bei gesetztem Monoflop 61 über ein UND-Gatter 68 und ein ODER-Gatter 69 zum Rücksetzeingang des Flip-Flops 64, so daß der Zählvorgang unterbrochen wird. Einige Millisekunden verzögert wird vom Ausgang des UND-Gatters 68 her über ein Verzögerungsglied 70 der Eingang "Auslesen" des Zählers 67 aktiviert, so daß der ermittelte Zählerstand in den nachfolgenden Mittelspeicher 7 überführt wird. Nach Ablauf der Impulsdauer des Monoflops 61 wird nach Usetzung der Rückflanke in einen Steuerimpuls in einem Impulswandler 72 über ODER-Gatter 73 und 74 das Flip-Flop 64 und der Zähler 67 zurückgesetzt, ohne daß ein Auslesen erfolgt. Mittels eines weiteren Monoflops 74, das in die "Setz"-Leitung des Flip-Flops 64 eingefügt ist, wird eine Auswertung der systolischen Zeitintervalle in schnellerer Folge als es der normalen Herzrate entspricht, verhindert, da ein UND-Gatter 75 im Eingang des Monoflops über seinen invertierenden Eingang gesperrt ist, solange dessen Impuls nicht abgeklungen ist. Die Impulsdauer des Monoflops 74 entspricht dabei dem maximalen zu verarbeitenden RR-Abstand der entsprechenden Herzfrequenz.

Der Mittelwertspeicher 7 enthält einen Arithmetikteil 71, der die bisher erfaßten systolischen Intervalle (hier: LVET) mit schwerpunktmäßiger Berücksichtigung der letzten fünf Ereignisse ausmittelt und diesen Wert festhält. Bei Änderung des Betriebszustands bzw. einem Übergang vom stimulierenden Betrieb zu Ruhezustand des Schrittmachers bei vorhandenem Sinusrhythmus wird der Arithmetikteil 71 des Mittelwertspeichers gelöscht, so daß mit den nachfolgend aufgenommenen systoliscben Intervallen erneut mit der Mittelwertbildung begonnen wird. Ein Auslesen aus dem Mittelwertspeicher erfolgt mit der Erfassung jeweils eines neuen Mittelwertes, nachdem dieser verarbeitet wurde. (Ausgang: Ansteuerung Rechner und Aktivierung der Ausgabe des Arithmetikteil). Um die Häufigkeit der Veränderung der Stimulationsfrequenz zu verringern, kann hier aber auch eine Herabsetzung der Schaltfrequenz durch Herunterteilen erfolgen. Ein Zähler 76 sperrt mittels eines nachgeschalteten UND-Gatters 77 die Ausgabe von ermittelten Intervallwerten nach einem Wechsel des Betriebszustands, da der Zähler in diesem Fall zurückgesetzt wird (Eingang "Reset") und er ein das UND-Gatter 77 durchschaltendes Ausgangssignal erst dann abgibt, wenn der Zählerstand $\geq 5$ ist.

Bei der zuvor dargestellten Einspeicherung der aktuellen systolischen Intervalle bei Spontanaktionen erfolgt die Steuerung aufgrund der abgespeicherten Werte bei der Stimulation unter der Kontrolle eines zusätzlich vorgesehenen Rechners 12, der ausgelöst durch ein neues im Herzen aufgenommenes Intervallsignal, die zugehörige Grundrate im Speicher 11 aufgrund zusätzlicher Informationen und über die Kommunikationseinheit 3 eingegebener Vorgaben zusätzlich verändern kann, wobei Zugriff auf den Speicher 9 besteht. Diese Veränderung besteht insbesondere in der Anbringung eines Korrekturwertes, welcher bei der Steuerung der Grundrate des Schrittmachers unter Stimulationsbedingungen und Auswertung der entsprechenden Intervalle bei Spontanaktionen, die sich aufgrund der Änderung dieser Bedingungen für das Herz ergebenden Änderung der systolischen Intervalle berücksichtigt. Weiterhin kann eine Interpolation oder ein Ausgleich bei sich ergebenden Frequenzsprüngen bei benachbarten Speicherplätzen ausgeglichen werden. Außerdem lassen sich über die Kommunikationseinheit 3 programmierte Grenzwerte 18 für die Stimulationsfrequenzen vorgeben, welche bei der Nachführung der Grundrate des Schrittmachers nicht überschritten werden. Der entsprechende Grenzwertspeicher wird vom Rechner gelesen und bei Überschreitung des entsprechendes Wertes durch die Steuerung aufgrund der systolischen Intervalle eine Korrektur des im Grundratenspeicher 11 übernommenen Frequenzwertes bewirkt.

Auch die übrigen angegebenen Betriebsweisen stellen Speicheroperationen im Hinblick auf den Speicher 9 dar, wobei in dem matrixartig organisiertem Speicher in Abhängigkeit von den vorliegenden Betriebsfällen jeweils bei den einzelnen Speicherplätzen in Abhängigkeit von dem physiologischen Parameter Zeitwerte für die Escape-Intervalle abgelegt oder abgefragt werden. Dieses Ablegen oder Abfragen erfolgt dabei gegebenenfalls für die Betriebszustände "stimulierte Kammeraktionen" und "Spontanaktionen" in separaten, aber im übrigen parallel adressierbaren Speicherplätzen. Entsprechend läßt sich eine darartige Unterscheidung der beiden Betriebszustände bei zugeordneten festen Differenzwerten der Parameter auch durch eine entsprechende Beeinflussung der parametergesteuerten Adressierung bewirken. Im Falle der Ausnehmung bestimmter Intervallbereiche, werden in den den betreffenden Parametern zugeordneten Speicherplätzen diejenigen Intervallwerte eingegeben, welche statt dessen gültig sind — also die Grenzintervallwerte der im Stimulationsbetrieb "ausgenommenen" Frequenzbereiche.

In Figur 1a ist der aus den Bausteinen 4 bis 7 bestehende Eingangsteil der Schaltung im Detail wiedergegeben. Dabei besteht die im Mikrofon 4 nachgeschaltete Verarbeitungsgruppe aus einem Eingangsverstärker 51 zur Heraufsetzung der Eingangssignale, einem Filter 52 zur Ausfilterung der für die systolischen Zeitintervalle charakteristischen Frequenzbereiche, einem Schmitt-Trigger 53, einem Gleichrichter und Siebglied 53, der Umwandlung der Frequenzsignale in Impulse einer Polarität, einem Schmitt-Trigger als Schwellwertdiskriminator und einem Impulsformer 55 zur Umwandlung der Vorderflanke des Ausgangsimpulses der Schaltung 54 in einen kurzen Steuerimpuls.

In Figur 2 ist in der Draufsicht ein Steuerteil 200 für die externe Programmierung und Kontrolle für die Hand des Arztes wiedergegeben. Auf dem Display 201 in der linken Bildhälfte wird die funktionale Abhängigkeit der Herzfrequenz in Abhängigkeit von den zu erfassenden systolischen Intervallen dargestellt, wobei die Wiedergabe jeweils für verschiedene Betriebszustände getrennt (oder überlagert) vorgenommen wird. Die Darstellung entspricht den im Speicher 9 des Schrittmachers festgehaltenen Werten, wobei entweder ein fester funktionaler Zusammenhang vorgegeben werden kann, oder aber die für die im implantierten System ermittelten systolischen Intervalle einzuhaltenden Stimulationsfrequenzen aufgrund der bei vorhandenem Sinusrhythmus aufgefundenen Werten errechnet werden. Auch lassen sich bei vorbestimmten Bereichen der Werte der systolischen Intervalle bevorzugt einzuhaltende Stimulationsfrequenzen einprogrammieren sowie Differenzen der Frequenzen welche zu Intervallwerten bei unterschiedlichen Betriebsarten gehören, um diese Korrekturwerte beim Wechsel des Modes etc. heranziehen zu können. Die Verarbeitung der Herz- und Stimulationsfrequenzen erfolgt in Form des (reziproken) RR-Intervalls, um hinsichtlich der Verarbeitung proportionalität zu den erfaßten Intervallzeiten zu erhalten und inverse Zusammenhänge zui vermeiden.

Der in Figur 3 dargestellte Schallempfänger läßt sich sowohl im Schrittmacher selbst als auch in der im Herzen verankerten Elektrode unterbringen. Er enthält einen einseitig eingespannten Piezowandler 301, der über ein mechanisches Koppelelement 302 mit einer eine das Gehähse bzw. die Wandung 303 abschließenden Membran 304 in Verbindung steht.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen. Insbesondere beschränkt sich die Ausführung nicht auf die Realisierung mit diskreten logischen Baugruppen, sondern läßt sich vorteilhaft auch mit programmierter Logik — vorzugsweise unter Verwendung eines Mikroprozessors — realisieren.

**Patentansprüche**

1. Herzschrittmacher mit Mitteln zur Steuerung der Zeitpunkte der Stimulationsimpulse mittels mindestens eines innerhalb des Körpers aufgenommenen physiologischen Parameters, dadurch gekennzeichnet, daß die Stimulations-Intervalle für vorgegebene Werte des Parameters im wesentlichen derart eingestellt sind, daß sie den Zeiten zwischen aufeinanderfolgenden gleichartigen Herzaktionen entsprechen, welche zuvor bei zwei im natürlichen Herzrhythmus auftretenden, Spontanaktionen für übereinstimmende Werte des Parameters ermittelt wurden.

2. Herzschrittmacher nach Anspruch 1, dadurch gekennzeichnet, daß Speichermittel vorgesehen sind, welche bei auftretenden Spontanaktionen eine aktuelle Rate in Zuordnung zu dem aktuellen physiologischen Parameter festhalten und beim Ausfall der Spontanaktionen Stimulationsimpulse mit einer Rate abgeben, die dem dann vorhandenen Parameterwert zugeordnet ist.

3. Herzschrittmacher nach einem der vorangehenden Ansprüche, dadurch gekennzeichnet, daß es sich im Falle eines Demand-Schrittmachers bei den Stimulationsintervallen um die Escape-Intervalle handelt.

4. Herzschrittmacher nach einem der Ansprüche 2 bis 3, dadurch gekennzeichnet, daß die Stimulationsrate, die einem vorgegebenen Parameterwert zugordnet ist, niedriger gewählt ist als die sich bei diesem Parameterwert einstellende natürliche Stimulationsrate, also das Escape-Intervall bezogen auf einen vorgegebenen Wert des Parameters jeweils um einen Zeitbetrag größer gewählt ist als der zugehörige Abstand zwischen gleichartigen Spontanaktionen.

5. Herzschrittmacher nach einem der vorangehenden Ansprüche, dadurch gekennzeichnet, daß bei einem Parameter, dessen aktueller Wert in Abhängigkeit von der Herzfrequenz auch von der Art Initiierung der Herzaktionen (spontan oder stimuliert) abhängt, das Escape-Intervall bei einer vorangehenden Spontanaktion in Abhängigkeit von Parameterwerten festgelegt wird, die dem Betrieb ohne Schrittmacherintervention entsprechen, während das Escape-Intervall bei einer vorangehenden stimulierten Aktion in Abhängigkeit von Parameterwerten festgelegt wird, die dem stimulierendem Betrieb entsprechen.

6. Herzschrittmacher nach Anspruch 5, dadurch gekennzeichnet, daß die Werte der Escape-Intervalle für die Stimulation auf vorangehende Spontanaktionen hin und für die Stimulation nach vorangehenden stimulierten Aktionen sich im wesentlichen durch eine feste additive Konstante für die Intervallzeit bzw. für den Parameterwert unterscheiden.

7. Herzschrittmacher nach Anspruch 5, dadurch gekennzeichnet, daß die Werte der Escape-Intervalle für die Stimulation auf vorangehende Spontanaktionen hin und für die Stimulation nach vorangehenden stimulierten Aktionen sich im wesentlichen durch eine veränderliche additive Konstante für die Intervallzeit bzw. bezüglich des Parameterwerts bei übereinstimmenden zu vergleichenden Escape-Intervallen unterscheiden.

8. Herzschrittmacher nach den Ansprüchen 6 oder 7, dadurch gekennzeichnet, daß eine Korrekturgröße für verschiedene Herzfrequenzen in einer Tabelle abspeicherbar ist.

9. Herzschrittmacher nach einem der Ansprüche 5 bis 8, dadurch gekennzeichnet, daß die Korrekturgröße entweder mittels externer Programmiermittel eingegebbar ist oder beim Übergang von spontanen zu stimulierten Aktionen bzw. umgekehrt in bezug auf den dabei aktuellen Parameter ermittelt wird.

10. Herzschrittmacher nach einem der vorangehenden Ansprüche, dadurch gekennzeichnet, daß die Anpassung des funktionalen Zusammenhangs zwischen der Herzfrequenz und dem Parameter bzw. der Korrekturgröße während des Schrittmacherbetriebs bei auftretenden Spontanaktionen selbsttätig aktualisiert wird.

11. Herzschrittmacher nach einem der vorangehenden Ansprüche, dadurch gekennzeichnet, daß der funktionale Zusammenhang zwischen physiologischen Parametern und Escape-Intervallen derart einstellbar ist, daß vorbestimmte Zeitbereiche dieser Intervalle durch den obern oder unteren Grenz-Intervallwert oder einem diesen benchbarten Wert ersetzt werden.

12. Herzschrittmacher nach Anspruch 11, dadurch gekennzeichnet, daß der jeweilige Bereich der Ersetzung in Abhängigkeit von der Richtung der Annäherung veränderbar ist, so daß sich ein Hystereseverlauf ergibt.

13. Herzschrittmacher nach einem der Ansprüche 11 oder 12, dadurch gekennzeichnet, daß die Ersetzung nur im Falle des Ausfalls von Spontanaktionen wirksam ist bzw. nur für diesen Fall wirksam schaltbar ist.

14. Herzschrittmacher nach einem der vorangehenden Ansprüche, dadurch gekennzeichnet, daß der Parameter durch mindestens ein systolisches Intervall, das QT-Intervall und/oder ein elektroplethysmographisches Signal gebildet wird.

**Revendications**

1. Stimulateur cardiaque avec des moyens pour commander les instants des impulsions stimulatrices au moyen d'au moins un paramètre physiologique capté à l'intérieur du corps, caractérisé par le fait que les intervalles de stimulation pour des valeurs prédéterminées du paramètre sont réglés sensiblement de manière à correspondre aux temps entre réactions cardiaques successives de même nature, qui ont été préalablement déterminées à l'occasion de deux actions spontanées survenant dans le rythme cardiaque naturel, pour des valeurs correspondantes du paramètre.

2. Stimulateur cardiaque selon revendication 1, caractérisé par le fait qu'il est prévu des moyens de mémoire qui, lors de l'occurrence d'actions spontanées, retiennent une cadence actuelle en correspondance avec le paramètre physiologique actuel et, en cas de défaillance des actions spontanées, fournissent des impulsions stimulatrices à une cadence qui est alors en correspondance avec la valeur existante du paramètre.

3. Stimulateur cardiaque selon l'une des revendications précédentes, caractérisé par le fait que, dans le cas d'un stimulateur "Demand", c'est des intervalles "Escape" qu'il s'agit pour ce qui est des intervalles de stimulation.

4. Stimulateur cardiaque selon l'une des revendications 2 à 3, caractérisé par le fait que la cadence de stimulation qui est associée à une valeur de paramètre donnée à l'avance est choisie plus basse que la cadence de stimulation naturelle s'établissant pour cette valeur de paramètre, l'intervalle "Escape" rapporté à une valeur préétablie du paramètre étant à chaque fois choisi plus grand, d'une valeur de temps, que l'intervalle y relatif entre actions spontanées de même type.

5. Stimulateur cardiaque selon l'une des revendications précédentes, caractérisé par le fait que, dans le cas d'un paramètre dont la valeur actuelle en fonction de la fréquence cardiaque dépend aussi du type d'initialisation des réactions cardiaques (spontanée ou stimulée), l'intervalle "Escape" dans le cas d'une action spontanée précédente est fixé en fonction de valeurs de paramètre qui correspondent à la marche sans intervention du stimulateur, tandis que l'intervalle "Escape" dans le cas d'une action stimulée précédente est fixé en fonction de valeurs de paramètre qui correspondent à la marche avec stimulation.

6. Stimulateur cardiaque selon revendication 5, caractérisé par le fait que les valeurs des intervalles "Escape" pour la stimulation sur actions spontanées précédentes et pour la stimulation après actions spontanées précédentes diffèrent essentiellement par une constante additive fixe pour le temps d'intervalle ou selon le cas pour la valeur de paramètre.

7. Stimulateur cardiaque selon revendication 5, caractérisé par le fait que les valeurs des intervalles "Escape" pour la stimulation sur actions spontanées précédentes et pour la stimulation après actions stimulées précédentes diffèrent essentiellement par une constante additive variable pour le temps d'intervalle ou selon le cas pour ce qui est de la valeur de paramètre en cas d'intervalles "Escape" concordants à comparer.

8. Stimulateur cardiaque selon les revendications 6 ou 7, caractérisé par le fait qu'une grandeur de correction pour différentes fréquences cardiaques peut être mémorisée dans une table.

9. Stimulateur cardiaque selon l'une des revendications 5 à 8, caractérisé par le fait que la grandeur de correction peut être introduite au moyen de moyens programmeurs externes ou lors de la transition d'actions spontanées à des actions stimulées, ou selon le cas est, inversement, établie par référence aux paramètres qui sont alors actuels.

10. Stimulateur cardiaque selon l'une des revendications précédentes, caractérisé par le fait que l'adaptation de la corrélation fonctionnelle entre la fréquence cardiaque et le paramètre ou la grandeur de correction pendant la marche du stimulateur est actualisée automatiquement lors de l'occurrence d'actions spontanées.

11. Stimulateur cardiaque selon l'une des revendications précédentes, caractérisé par le fait que la corrélation fonctionnelle entre paramètres physiologiques et intervalles "Escape" est réglable de manière telle que des plages de temps prédéterminées de ces intervalles soient remplacées par la valeur-limite d'intervalle supérieure ou inférieure, ou par une valeur voisine de celles-ci.

12. Stimulateur cardiaque selon revendication 11, caractérisé par le fait que la plage de remplacement concernée est modifiable en fonction du sens de l'approche, de sorte que l'on obtient une allure d'hystérésis.

13. Stimulateur cardiaque selon l'une des revendications 11 ou 12, caractérisé par le fait que le remplacement n'est effectif qu'en cas de défaillance d'actions spontanées, ou encore ne peut être rendu actif que pour ce cas.

14. Stimulateur cardiaque selon l'une des revendications précédentes, caractérisé par le fait que le paramètre est constitué par au moins un intervalle systolique, l'intervalle QT, et/ou par un signal électropléthysmographique.

## Claims

1. A cardiac pacemaker having means for controlling the moments of the stimulation pulses by means of at least one physiological parameter picked up inside the body, characterised in that the stimulation intervals for preset values of the parameter are essentially set in such a manner that they correspond to the times between successive similar heart actions, which have been previously determined during two spontaneous actions occurring in the natural rhythm of the heart for coinciding values of the parameter.

2. A cardiac pacemaker according to Claim 1, characterised in that storage means are provided which, on the occurrence of spontaneous actions, record an actual rate in coordination with the actual physiological parameter and in the event of non-appearance of the spontaneous actions, deliver stimulation pulses at a rate which is coordinated with the parameter value then present.

3. A cardiac pacemaker according to any one of the preceding Claims, characterised in that, in the case of a demand pacemaker, the stimulation intervals are the escape intervals.

4. A cardiac pacemaker according to any one of Claims 2 to 3, characterised in that the stimulation rate which is associated with a preset parameter value is selected lower than the natural stimulation rate becoming established at this parameter value, that is to say the escape interval is selected greater in relation to a preset value of the parameter by an amount of time in each case than the corresponding interval between similar spontaneous actions.

5. A cardiac pacemaker according to any one of the preceding Claims, characterised in that in the case of a parameter, the actual value of which, depending on the heart rate, also depends on the nature of initiation of the heart actions (spontaneous or stimulated), the escape interval is determined, in the event of a preceding spontaneous action, depending on parameter values which correspond to the operation without pacemaker intervention whereas in the event of a preceding stimulated action, the escape interval is determined depending on parameter values which correspond to the stimulating operation.

6. A cardiac pacemaker according to Claim 5, characterised in that the values of the escape intervals for the stimulation following preceding spontaneous actions and for the stimulation after preceding stimulated actions differ essentially by a fixed additive constant for the interval time or for the parameter value.

7. A cardiac pacemaker according to Claim 5, characterised in that the values of the escape intervals for the stimulation following preceding spontaneous actions and for the stimulation after preceding stimulated actions differ essentially by a variable additive constant for the interval time or with regard to the parameter value with corresponding escape intervals to be compared.

8. A cardiac pacemaker according to Claim 6 or 7, characterised in that a correction quantity for various heart rates can be stored in a table.

9. A cardiac pacemaker according to any one of Claims 5 to 8, characterised in that the correction quantity can either be fed in by means of external programming means or is determined, on the changeover from spontaneous to stimulated actions or vice versa with reference to the actual parameter at the time.

10. A cardiac pacemaker according to any one of the preceding Claims, characterised in that the matching of the functional relationship between the heart rate and the parameter or the correction quantity during operation of the pacemaker is updated automatically on the appearance of spontaneous actions.

11. A cardiac pacemaker according to any one of the preceding Claims, characterised in that the functional relationship between physiological parameters and escape intervals is adjustable in such a manner that predetermined time domains of these intervals are replaced by the upper or lower limiting interval value or a value close to this.

12. A cardiac pacemaker according to Claim 11, characterised in that the particular domain of the replacement is variable depending on the direction of the approach so that a hysteresis curve results.

13. A cardiac pacemaker according to Claim 11 or 12, characterised in that the replacement is effective only in the case of the non-appearance of spontaneous actions or can only be effectively operated for this case.

14. A cardiac pacemaker according to any one of the preceding Claims, characterised in that the parameter is formed by at least one systolic interval, the Q.T. interval and/or an electro-plethysmographic signal.

Fig.1

1

Betriebsartwechsel

**4**   **5**                      **6**                                **7**

51  52  53  54  55        62    61    63   75        64  65  67  71

D  MF        MF    SET Q       Takt    Res.
                              RES.     Res.  Ausles.

66  Takt

69  74

68        70   D                            Rech-
                                            ner

72        73

77
Takt
Res.
76

EP 0 203 027 B1

Fig. 1a

Fig. 2

Fig. 3